# EUROPEAN PATENT SPECIFICATION

(11) **EP 3 423 430 B1**
(45) Date of publication and mention of the grant of the patent: **08.04.2020**
(21) Application number: 17707013.3
(22) Date of filing: 24.02.2017
(51) Int. Cl.: C07C 211/14, C07C 209/48, C07C 253/30, C07C 255/24, C10M 133/06, C08G 73/02, C10L 1/222

(54) **DIALKYL-POLYALKYLAMINE COMPOSITIONS, PROCESS FOR THEIR PREPARATION AND THEIR USE**
DIALKYLPOLYALKYLAMIN-ZUSAMMENSETZUNGEN, VERFAHREN ZU DEREN HERSTELLUNG UND IHRE VERWENDUNG
COMPOSITIONS DIALKYL-POLYALKYLAMINE, PROCÉDÉ POUR LEUR PRÉPARATION ET LEUR UTILISATION

(30) Priority: 29.02.2016 US 201662300992 P; 18.03.2016 EP 16161213
(43) Date of publication of application: 09.01.2019
(73) Proprietor: Nouryon Chemicals International B.V., 6824BM Arnhem (NL)
(72) Inventor: OSKARSSON, Hans, 444 41 Stenungsund (SE); BOWEN, Lynnette Susan, Heswall Merseyside CH60 8QN (GB); JANIAK, John André, 417 21 Göteborg (SE); BAND, Elliot Isaac, Pleasantville, New York 10570 (US); RUS, Kristan Alexander, 3816 TR Amersfoort (NL)
(74) Representative: LKGlobal UK Ltd.
(86) International application number: PCT/EP2017/054281
(87) International publication number: WO 2017/148808

(56) References cited:
- WO-A1-2006/076929
- FR-A1- 2 539 754
- FR-A1- 2 834 715
- US-A1- 2003 049 310
- US-B1- 6 667 382
- PIERI, C. ET AL.: "First evidence for the use of polyamines as nucleophiles in a regioselective palladium-catalyzed allylic amination reaction", TETRAHEDRON, vol. 70, no. 51, 12 October 2014 (2014-10-12), pages 9718-9725, XP55365027, ISSN: 0040-4020, DOI: 10.1016/j.tet.2014.10.019

## Description

### Field of the invention

The present invention relates to compositions of di-fatty-alk(en)yl polyalkylamines whereof the polyalkylamine-moiety shows a specific level of branching, a process to make such compositions, and their use in various applications.

### Background of the invention

Alk(en)yl polyalkylamines are well known and are used, inter alia, in asphalt applications.

WO 2006/076929 discloses, as intermediates in the synthesis of fully tertiary mono-fatty-alkyl polyalkylamines, a fully linear product with one fatty alkyl moiety. Although the reaction pathway is mentioned, the reaction conditions are not clear and the products, that have only one fatty alkyl moiety, seem to be pure. Salts of the fully tertiary mono-fatty-alkyl triamines are mentioned to be suitable for use in aqueous slow-setting bitumen-aggregate mixtures.

US 4,967,008 relates to mono-fatty-alkyl polyalkylamines with primary, tertiary and optionally secondary amine moieties. An exemplified product is linear N-tallow-N-methyl dipropylene triamine. Because the intermediate in the process of US4967008 is methylated before the second cyanoethylation step, there is no branching in the products.

From CAS registration number 1623405-26-4, a linear di-fatty-alkyl tetramine with CAS-name "Amines, N'-{3-[(3-aminopropyl)amino]propyl}-N,N-di-C16-18 alkyltrimethylenedi-" is known.

US2003/049310 discloses a large amount of cationic lipids and the use thereof in liposomes to introduce functional bioactive agents to cultures cells. Mixtures of polyamines as claimed are not disclosed or suggested.

WO 2006/076929, US 6,667,382, and FR 2,834,715 all disclose specific polyamine compositions that are not the same as the mixture of products as claimed.

For lubricating oils a variety of amines have been evaluated in the past. Particular for 2-stroke marine engines it is known that due to changes in emission regulations, as well because of the different fuels used, there is a need for an improved amine formulation for use in lubricating oils, particularly for use in lubrication oils used in engines that burn sulfur-containing oils, particularly for lubrication of 2-stroke diesel marine engines.

Surprisingly it was found that compositions of di-fatty-alk(en)yl tetramines with a specific amount of linear and branched molecules show excellent properties when used in lubrication oils and other applications where polyalkylamines are known to be used, such as in demulsifiers for oil-in-water emulsions, generic use as a corrosion inhibitor, fuel additive, anti-scaling agent, asphalt additive, enhanced oil recovery from oil-wells, cutting-oil additive, and anti-static agent. Furthermore, it was found that the claimed processes to make the di-fatty-alk(en)yl tetramines is very economic in producing the desired compositions.

Accordingly, the invention relates to such di-fatty-alk(en)yl polyalkylamine compositions which comprise mixtures of one or more polyalkylamines selected from products of the formulae (I) and (II) 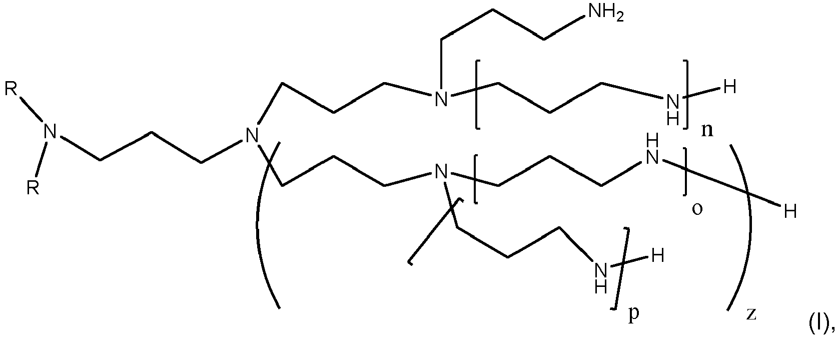 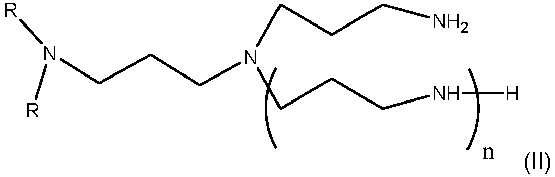 wherein each R is, independent of the other R, a fatty-alkyl or fatty-alkenyl moiety, herein defined as to have 8-22 carbon atoms, which is linear or branched, n and z are independent of each other either 0, 1, 2, or 3, and when z>0 then o and p are independent of each other either 0, 1, 2, or 3, or methylated and/or alkoxylated derivatives thereof, whereby said mixture comprises at least 3 % by weight of branched products whereof at least one of n and z >= 1.and whereby said mixture comprises at least 5 % by weight of products with a pure linear structure. Such mixtures with both linear and branched products were found to have a desirable viscosity profile.

In an embodiment the amount of products with linear structure in the mixture is 6, 7.5, 10, or 14 % by weight, or more, based on the weight of all polyalkylamines. In an embodiment the linear product in the mixture is of formula (I) with n and z being 0. In an embodiment the linear product in the mixture is of formula (II) with n is 0.

In an embodiment the mixtures comprise at least 4 % by weight (%w/w), suitably at least 5 %w/w, suitably at least 6 %w/w, suitably more than 7 %w/w, suitably more than 7.5 %w/w, suitably more than 10%w/w, suitably more than 20%w/w, of branched compounds whereof, for the products of formula (I), at least one of n and z >= 1, based on the weight of all polyalkylamines. For the products of formula (II) this means that for the branched products n must be >=1.

It is noted that whenever n, o, p, or z is 0 then the hydrogen is covalently bound to the nitrogen. For economic reasons each of n, o, p, and z, independently and when not 0, are 1 or 2, preferably 1.

Although the two R groups can be different, they are, in one embodiment, the same, since such materials are more economically produced. Irrespective of whether they are the same or not, one or both of the R groups, independently, are typically derived from chemical feedstock or from a natural source, such as from natural oils and fats. Particularly if a natural source is used, it means that each R group may have a certain distribution in the carbon chain length. Suitably R is derived from animal and vegetal oils and fats, such as tallow, coco and palm oil. Since making the di-fatty-alkyl(ene) polyalkylamines in accordance with the invention comprises a hydrogenation step, it may be beneficial to use hydrogenated R groups before the products of the invention are prepared using a process as claimed. However, for certain feedstocks, even after hydrogenization, an appreciable amount of unsaturated bonds may remain. Suitably a fully hydrogenated tallow group is used as the R group, and the corresponding mixture of di-fatty-alkyl polyalkyl amines are formed. Alternatively, the raw material for the R group is unsaturated whereby the unsaturated R group may be wholly or partially hydrogenated during the process to make the claimed di-fatty-alk(en)yl polyalkylene amines being a mixture of di-fatty-alkyl polyalkylene amines and di-fatty-alkenyl polyalkylene amines. Also products with one fully saturated R group and one unsaturated R group are products of the invention.

Therefore, as used herein, di-fatty-alk(en)yl polyalkylamines" refers to di-fatty-alkyl polyalkylamines, di-fatty-alkenyl polyalkylamines, fatty-alkyl fatty alkenyl polyalkylamines, and mixtures thereof.

Where upper and lower limits are quoted for a property, for example for the concentration of a component, then a range of values defined by a combination of any of the upper limits with any of the lower limits may also be implied.

Derivatives of the di-fatty-alk(en)yl polyalkylamines compositions of the invention include products wherein one or more of the NH moieties of the dialkyl polyalkylamines of the invention are methylated, alkoxylated, or both. Such products were found to have desirable solubility of the dialkyl polyalkylamines, particularly in lubricating oils. Alkoxylated derivatives are suitably butoxylated, propoxylated and/or ethoxylated. If two or more different alkoxylation agents are used then can be used in any sequence, e.g. EO-PO-EO, and the various alkoxy units can be of blocky nature and/or be present in a random fashion. Suitably a primary -NH₂ group is alkoxylated with one or more alkylene oxides in a conventional way to form a -NH-AO-H group, wherein AO stands for one or more alkylene-oxy units. The resulting -NH-AO-H group can be further alkoxylated to form -N (AO-H)₂ groups. Especially when large amounts of alkylene oxide (i.e. when more than 8 AO molecules per polyalkylamine molecule) are used, typically also one or more of the secondary amine functions, if present, are alkoxylated. In an embodiment all primary and secondary amine functions of the di alkyl polyamine are alkoxylated. In another embodiment the di-fatty-alk(en)yl polyalkylamines are derivatized by methylating one or more of the N-H functions in a conventional way, for example by reaction with formic acid and formaldehyde. In another embodiment one or more of the O-H functions of an alkoxylated di-fatty-alk(en)yl polyalkylamines is methylated in a conventional way.

Although the inventors do not wish to be bound by the following theory, it is believed that the beneficial properties of the dialkyl polyalkylamines compositions as claimed, when compared to the fully branched or fully linear products, lie in the special interaction of the amines in the composition. More specifically, the mixture of linear and branched products are believed to be the components for achieving the desired combination of rheological and anticorrosion properties of the fluids in which the composition is used.

In view of this theory, it can be preferred to have products with a multitude of differently branched molecules. In that case, compositions comprising mixtures of polyalkylamines of the formula (I) are preferred. However, since compositions comprising mixtures of polyalkylamines of the formula (II) can be more economical to make, under specific circumstances compositions comprising mixtures of polyalkylamines of the formula (II) may be preferred. If suitable, compositions comprising mixtures of dialk(en)yl polyalkylamines of the formulae (I) and (II) are used.

In one embodiment the invention relates to the use of a composition comprising both the branched products in combination with a linear product according to formula 1 wherein n and z are 0, which composition was found to also show the desired rheology and properties.

The claimed compositions are suitably used as demulsifier for oil-in-water emulsions, corrosion inhibition, fuel additive, anti-scaling agent, asphalt additive, enhanced oil recovery agent from oil-wells, cutting-oil additive, anti-static agent, and additive in lubricating oils, particularly in lubricating oils for machines with varying amounts of acidic, specifically sulfuric acid, contaminants. One particular interesting field of use is in 2-stroke marine diesel engines which are typically operated with different fuels, each with different sulfur content, depending on availability, price, and environmental regulations.

The dialk(en)yl polyalkylamines mixtures as claimed can be produced using conventional process steps that are conducted in such order and way that the claimed mixtures are obtained. A suitable way to produce them is described in the experimental section below starting from a diamine and involving two or more cycles, for economic reasons preferably two, with each a cyanoethylation step and a hydrogenation step, hereinafter the two-cycle process. However, an alternative process wherein one equivalent of a di-alkyl-diamine is reacted in one step with two or more equivalents of acrylonitrile followed by hydrogenation, and optional further cycles involving a cyanoethylation and hydrogenation step, can be beneficial since it requires less reaction steps.

For increased branching in the two-cycle process, an acidic catalyst is used, such as HCI or acetic acid. Also increasing the reaction temperature during cyanoethylation will result in increased branching in this process. In an embodiment of a multicycle process, the temperature of a later cyanoethylation step is higher than the temperature in an earlier cyanoethylation step, to get a product with the desired branching. In an embodiment more than 1 mole of acrylonitrile is used per mole of the starting polyamine, which was also found to increase the branching of the resulting product to the desired level.

The temperature in each cyanoethylation step is suitably selected in the range from 70 to 125 °C. In an embodiment the reaction is conducted, for economic reasons, at a temperature up to 80, 85, 90, 95, or 100 °C.

For maintaining a homogeneous reaction mixture a solvent is suitably used. Suitable solvents include C₁₋₄ alcohols and C₂₋₄ diols. Ethanol may be a solvent of choice for ease of handling. Surprisingly the C₁₋₄ alcohols and C₂₋₄ diols were found not to be mere solvents. They turned out to also have co-catalytic activity in the cyanoethylation step.

The amount of solvent to be used can vary over a wide range. For economic purposes, the amount is typically kept at a minimum. The amount of solvent, particularly in a cyanoethylation step, is suitably less than 50, 40, 30, or 25 % by weight of the liquid reaction mixture. The amount of solvent, particularly in a cyanoethylation step, is suitably more than 0.1, 0.5, 1, 5, or 10 % by weight of the liquid reaction mixture.

The di-fatty-alk(en)yl polyalkylamines compositions of the invention were found to be particularly suitable as a corrosion inhibitor, particularly in cutting-oils and lubricating oils. However, they are also suitably used as a demulsifier for oil-in-water emulsions, a fuel additive, an anti-scaling agent, an asphalt additive, an enhanced oil recovery agent for oil-wells, and as an anti-static agent.

### EXPERIMENTAL

Duomeen® 2HT is available from AkzoNobel.

Other chemicals were sourced from SigmaAldrich, unless indicated differently.

### Example 1

A fully branched product with 4 amine functions was prepared using a 1L glass reactor with turbine stirrer to which chemicals can be dosed using a Prominent Gamma/L membrane pump and which was thermostatted using a Lauda K6KP heating bath.

### Raw materials

| Chemical | Supplier | Intake | Molw. | Intake |
|---|---|---|---|---|
| | | (g) | (g/mol) | (mol) |
| Duomeen 2HT | AkzoNobel | 342.0 | 566 | 0.604 |
| Hydrochloric acid (36%) | JT Baker | 3.06 | 36.5 | 0.014 |
| Water | Tap | 1.12 | 18.0 | 0.062 |
| Isopropanol | JT Baker | 34.2 | 60.1 | 0.569 |
| Sodium carbonate | Acros | As needed, see text | | |
| Acrylonitrile | Acros | 81.5 | 53.1 | 1.299 |
| Raney Cobalt | CatAlloy | As needed, see text | | |
| Ammonia | Air products | As needed, see text | | |

### Procedure & Results

The cyano-ethylation step is performed by charging the reactor with Duomeen 2HT, isopropanol (co-catalyst and solvent for the dicyano-product that is formed), water, and HCI, and subsequent dosing, in approximately three hours, of the acrylonitrile. Reaction pathway: 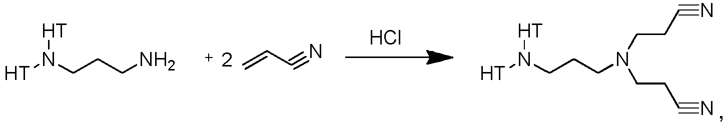 wherein HT stands for hydrogenated tallow.

After a conversion of 80% the reaction rate was so slow that the reaction was stopped. Vacuum was applied to the reactor the temperature was increased to 110°C to remove the acrylonitrile, water & IPA. The product was washed and neutralized in two steps with 4% Na₂CO₃ solution to remove all HCI, and subsequently hydrogenated using the same equipment. Reaction pathway:

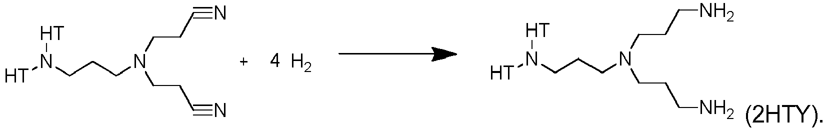

Thereto the stirred reactor containing the dicyano-product was charged with a conventional Raney Cobalt catalyst, such as A-7000 ex Johnson Matthey or Acticat®1100 ex CatAlloy, and subsequently heated to 130 °C while sparging with nitrogen, to remove traces of acrylonitrile and water. Then the reactor was charged with ammonia (13-14 barg) while kept at a temperature of 105 °C. Then the reactor was heated to 150 °C, and hydrogen was added to maintain a pressure of 49 barg. After completion of the reaction, the temperature was lowered to 80 °C and remaining hydrogen and ammonia were flushed out using nitrogen.

The resulting composition was analyzed using GC-MS and found to contain >70% of the product 2HTY of formula (II) with n=1, as well as more than 14%w/w of the linear product (HT)₂N-(CH₂)₃-NH-(CH₂)₃-NH₂, a little starting product (HT)₂N-(CH₂)₃-NH₂, and some unidentified further alkylamines.

### Example 2a and 2b

A mixture of linear and branched product (Tetrameen 2HTb) was prepared by the two cycle procedure wherein the cyano-ethylation and the hydrogenation steps above were repeated. The 0.6 mole of the Duomeen 2HT was combined with 0.65 mole of acrylonitrile and reacted in the first cyano-ethylation step. After hydrogenation, the triamine was combined with another 0.65 mole of acrylonitrile and reacted. At the end of each cyano-ethylation step, NMR was used to analyze the reaction mixture and to determine if one mole of acrylonitrile had reacted per mole of starting material. If the reaction was found to be too low, some additional acrylonitrile was dosed and after 1 hour the analysis was repeated. This cycle was repeated till the desired reaction was obtained. The final product was analyzed using GC-MS applying the following conditions

| | | |
|---|---|---|
| Gas chromatograph | TRACE ULTRA GC Interscience | |
| MS system | ISQ GC-MS | |
| Column | Fused silica WCOT, 20 m x 0.32 mm ID | |
| - stationary phase | Sil 5 CB, 100% polydimethyl-siloxane, cross-linked | |
| - film thickness | 0.12 µm | |
| Carrier gas | Helium | |
| - flow | 2 ml/min. | |

| Temperatures | | |
|---|---|---|
| - injector | 275°C | |
| - column | initial | : 200 °C during 1 min |
| | rate : | 20 °C/min |
| | final | : 310 °C during 15 min |
| Injection volume | 1 µl, approx. 250mg sample in 10 ml cyclohexane | |

In the examples no additional acrylonitrile had to be added after the first cyanoacrylation step which was conducted at a temperature of 85 °C and 75 °C, for examples 2a and 2b respectively. In the second cyanoacrylation step the temperature was 85 and 80 °C, for examples 2a and 2b respectively. In example 2a an additional amount of 0.025 mole of acrylonitrile was needed to complete the second cyanoacrylation step while in example 2b an additional amount of 0.12 mole of acrylonitrile was added before the addition of 0.60 mole of acrylonitrile was achieved. The highest amount of branching was observed in the sample 2a that was highest in temperature.

It was confirmed that the off-white products, which were pasty/viscous liquids at room temperature, contained more than 13.8%w/w of branched product of formula (I) with one or more of n and z >=1, and also contained more than 14%w/w of the linear product with n=z=0.

### Examples 3-5

To a commercial lubricant (Talusia HR70, which is overbased and comprises CaCO₃, from Total LubeMarine), 5wt% of the 2HTb of example 2a, 2HTY, or a 50/50 blend of 2HTY and 2HTb was added and thoroughly blended, followed by neutralization of 50 BN points of the lubricant composition with 95% sulfuric acid, in order to simulate the phenomenon of neutralization of the composition to be closer to real conditions of use of the lubricating composition in a marine engine.

In this process the amines together with the overbased detergents neutralizes the sulfuric acid. The generated sulfate ions become the counterions of the positively charged ammonium groups and/or react with the calcium of the CaCO₃ to form gypsum, CaSO₄.

Measurements of the viscosity (Pa.s) at 40°C of the three acidized blends of lubricant and alkylpolyamines, as prepared above, were performed by measuring the viscosity at a shear rate of 0.05 s-1 as displayed in the tables. All measurements were performed at 40 °C on an AR-G2 rheometer from TA-instruments.

**Table 1**

| Product | Viscosity |
|---|---|
| | Pa.s |
| 2HTb | 0.3639 |
| 2HTY | 0.3838 |
| 50/50 of 2HTb/2HTY | 0.3561 |

All of these samples show an acceptable viscosity.

### Comparative examples A-C

Acidized blends of a commercial mono-oleyl tripropylenetetramine of AkzoNobel (Tetrameen OV) and a commercial mono-tallow tripropylenetetramine of AkzoNobel (Tetrameen T) in lubricant, prepared as described for Examples 3-5, were analyzed for its viscosity and compared with the viscosity of the 2HTb blend and lubricant only.

**Table 2**

| Product | Viscosity |
|---|---|
| | Pa.s |
| Tetrameen OV | 0.8061 |
| Tetrameen T | 2.705 |
| 2HTb | 0.3639 |
| Base oil | 0.2695 |

The results show that Tetrameen OV and Tetrameen T resulted in an unacceptable viscosity of the acidized blend.

### Comparative examples D-F

Acidized blends of a commercial di-hydrogenated tallow dipropylenetriamine of AkzoNobel (Triameen® 2HT) and a commercial di-hydrogenated tallow propylenediamine of AkzoNobel (Duomeen® 2HT) in lubricant, prepared as described for Examples 3-5, whereby the amount of the triamine was increased to 6.2%w/w and the amount of Duomeen was increased to 8%w/w to achieve the same total base number, were analyzed for its viscosity and compared with the viscosity of the blends of comparative example A-C. The viscosity (Pa.s) at a shear rate of 0.05 s-1 was found to be between the viscosity for Tetrameen OV and Tetrameen T, so again showing an undesired high viscosity.

### Mental example 6

In this example the compounds of Examples 1, 2a and 2b are used as demulsifiers for oil-in-water emulsions, corrosion inhibitor, fuel additive, anti-scaling agent, asphalt additive, enhanced oil recovery agent for oil-wells, cutting-oil additive, and anti-static agent. They show the typical performance, but also a viscosity profile which is surprising for di-fatty-alk(en)yl polyalkylamines with this molecular weight.

## Claims

1. A polyalkylamine composition which comprises a mixture of polyalkylamines selected from products with formulae (I) and (II), 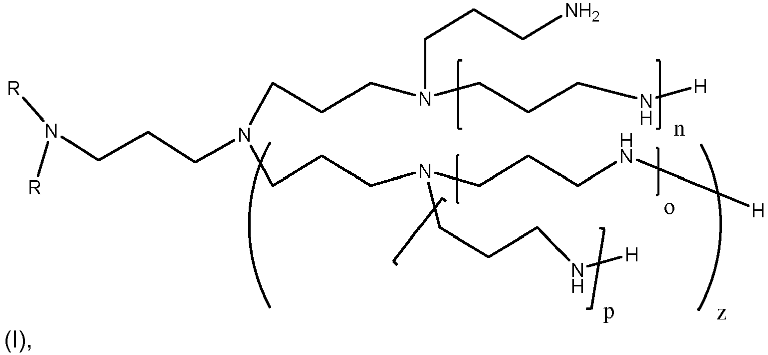 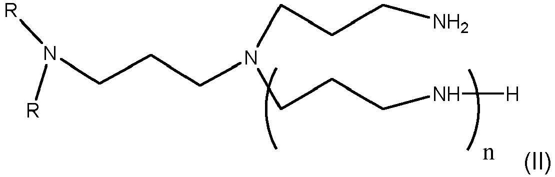 wherein each R is, independent of the other R, an alkyl or alkenyl moiety with 8-22 carbon atoms, n and z are independent of each other either 0, 1, 2, or 3, and when z>0 then o and p are independent of each other either 0, 1, 2, or 3, or methylated, and/or alkoxylated derivatives thereof, whereby said mixture comprises a) a total of at least 3 % by weight of branched compounds of formula (I) with at least one of n and z >= 1 and products of formula (II) with n >=1, and b) a total of at least 5 % by weight of products of formula (I) with n and z =0, and compounds of formula (II) with n = 0.

2. A polyalkylamine composition according to claim 1 comprising a total of at least 4 %w/w of branched compounds of formula (I) with at least one of n and z > 0 and compounds of formula (II) with n > 0.

3. A polyalkylamine composition according to claim 1 or 2 comprising at least 6% by weight of products of formulae (I) and (II) with a linear structure with n is 0 in formulae (I) and (II) and z is 0 in formula (I).

4. A polyalkylamine composition of any of the previous claims comprising derivatives of di-fatty-alk(en)yl polyalkylamines, wherein the derivatives are alkoxylates which are optionally methylated.

5. A polyalkylamine composition of any of the previous claims comprising derivatives of di-fatty-alk(en)yl polyalkylamines, wherein the derivatives are methylated.

6. A process for making polyalkylamine compositions of any one of the previous claims wherein a di-fatty-alk(en)yl alkyldiamine is reacted in two or more cycles whereby each cycle comprises a cyanoethylation step and a subsequent hydrogenation step.

7. A process of claim 6 wherein an acidic catalyst is used during the cyanoethylation steps.

8. A process of claim 6 or 7 wherein the reaction temperature during a later cyanoethylation step is higher than the temperature in an earlier cyanoethylation step.

9. A process of any one of claims 6-8 wherein more than 1 mole of acrylonitrile is used per mole of the starting polyamine.

10. A process for making the polyalkylamines compositions of any one of claims 1-5 wherein a di-fatty-alk(en)yl alkyldiamine is subjected to a cyanoethylation step and a subsequent hydrogenation step, whereby in the cyanoethylation step at least 2 mole of acrylonitrile is used per mole of di-fatty-alk(en)yl alkyldiamine and an acidic catalyst is used.

11. A process of claim 10 wherein the product is subjected to one or more further cycles comprising a cyanoethylation and subsequent hydrogenation step.

12. A process according to any one of claims 6-11 wherein one or more solvents selected from C₁₋₄ alcohol and C₂₋₄ diols is present in the cyanoethylation step.

13. A process of claim 12 wherein the amount of solvent is from 0.1 to 50 % by weight of the liquid reaction mixture.

14. A process to make the derivatives of claim 4 or 5, being the process of any one of claims 6-13, with an additional step wherein the di-fatty-alk(en)yl polyalkylamines are methylated, alkoxylated, or both.

15. Use of di-fatty-alk(en)yl polyalkylamine compositions of any one of claims 1-5 as demulsifiers for oil-in-water emulsions, corrosion inhibitor, fuel additive, anti-scaling agent, asphalt additive, enhanced oil recovery agent for oil-wells, cutting-oil additive, and anti-static agent.

## Patentansprüche

1. Polyalkylaminzusammensetzung, welche ein Gemisch von Polyalkylaminen, ausgewählt aus Produkten mit Formel (I) und (II), 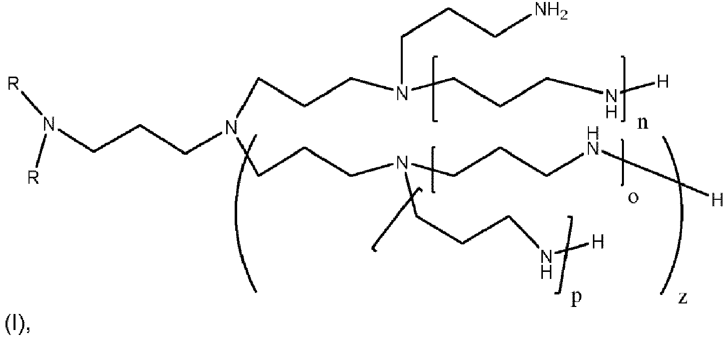 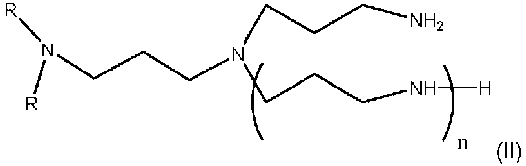 wobei jedes R unabhängig von dem anderen R ein Alkyl- oder Alkenyl-Molekülteil mit 8 bis 22 Kohlenstoffatomen ist, n und z unabhängig voneinander entweder 0, 1, 2 oder 3 sind und, wenn z > 0, dann o und p unabhängig voneinander entweder 0, 1, 2 oder 3 sind, oder methylierten und/oder alkoxylierten Derivaten davon umfasst, wobei das Gemisch a) insgesamt mindestens 3 Gewichts-% verzweigte Verbindungen mit der Formel (I) mit mindestens einem von n und z >= 1 und Produkte mit der Formel (II) mit n >= 1 und b) insgesamt mindestens 5 Gewichts-% Produkte mit der Formel (I) mit n und z = 0 und Verbindungen mit der Formel (II) mit n = 0 umfasst.

2. Polyalkylaminzusammensetzung nach Anspruch 1, umfassend insgesamt mindestens 4 Gew.-% verzweigte Verbindungen mit der Formel (I) mit mindestens einem von n und z > 0 und Verbindungen mit der Formel (II) mit n > 0.

3. Polyalkylaminzusammensetzung nach Anspruch 1 oder 2, umfassend mindestens 6 Gewichts-% Produkte mit der Formel (I) und (II) mit einer linearen Struktur, wobei n in Formel (I) und (II) 0 ist und z in Formel (I) 0 ist.

4. Polyalkylaminzusammensetzung nach einem der vorhergehenden Ansprüche, umfassend Derivate von Difettalk(en)ylpolyalkylaminen, wobei die Derivate Alkoxylate sind, die wahlweise methyliert sind.

5. Polyalkylaminzusammensetzung nach einem der vorhergehenden Ansprüche, umfassend Derivate von Difettalk(en)ylpolyalkylaminen, wobei die Derivate methyliert sind.

6. Verfahren zum Herstellen von Polyalkylaminzusammensetzungen nach einem der vorhergehenden Ansprüche, wobei ein Difettalk(en)ylalkyldiamin in zwei oder mehr Zyklen zur Reaktion gebracht wird, wobei jeder Zyklus einen Cyanoethylierungsschritt und einen nachfolgenden Hydrierungsschritt umfasst.

7. Verfahren nach Anspruch 6, wobei während der Cyanoethylierungsschritte ein saurer Katalysator benutzt wird.

8. Verfahren nach Anspruch 6 oder 7, wobei die Reaktionstemperatur während eines späteren Cyanoethylierungsschrittes höher als die Temperatur in einem früheren Cyanoethylierungsschritt ist.

9. Verfahren nach einem von Anspruch 6 bis 8, wobei mehr als 1 Mol Acrylnitril je Mol des Ausgangspolyamins benutzt wird.

10. Verfahren zum Herstellen der Polyalkylaminzusammensetzungen nach einem von Anspruch 1 bis 5, wobei ein Difettalk(en)ylalkyldiamin einem Cyanoethylierungsschritt und einem nachfolgenden Hydrierungsschritt unterworfen wird, wobei in dem Cyanoethylierungsschritt mindestens 2 Mol Acrylnitril je Mol Difettalk(en)ylalkyldiamin und ein saurer Katalysator benutzt werden.

11. Verfahren nach Anspruch 10, wobei das Produkt einem oder mehreren weiteren Zyklen, umfassend einen Cyanoethylierungs- und einen nachfolgenden Hydrierungsschritt, unterworfen wird.

12. Verfahren nach einem von Anspruch 6 bis 11, wobei ein oder mehrere Lösemittel, ausgewählt aus C₁₋₄-Alkohol und C₂₋₄-Diolen, in dem Cyanoethylierungsschritt vorhanden sind.

13. Verfahren nach Anspruch 12, wobei die Menge an Lösemittel 0,1 bis 50 Gewichts-% des flüssigen Reaktionsgemisches beträgt.

14. Verfahren zum Herstellen der Derivate nach Anspruch 4 oder 5, welches das Verfahren nach einem von Anspruch 6 bis 13 ist, mit einem zusätzlichen Schritt, wobei die Difettalk(en)ylpolyalkylamine methyliert, alkoxyliert oder beides werden.

15. Benutzung von Difettalk(en)ylpolyalkylamin-Zusammensetzungen nach einem von Anspruch 1 bis 5 als Demulgatoren für Öl-in-Wasser-Emulsionen, Korrosionshemmer, Kraftstoffzusatz, Kesselsteinverhütungsmittel, Asphaltzusatz, Mittel zur erhöhten Ölgewinnung für Ölbohrlöcher, Schneidölzusatz und Antistatikmittel.

## Revendications

1. Composition de polyalkylamines qui comprend un mélange de polyalkylamines sélectionnées parmi les produits répondant aux formules (I) et (II), 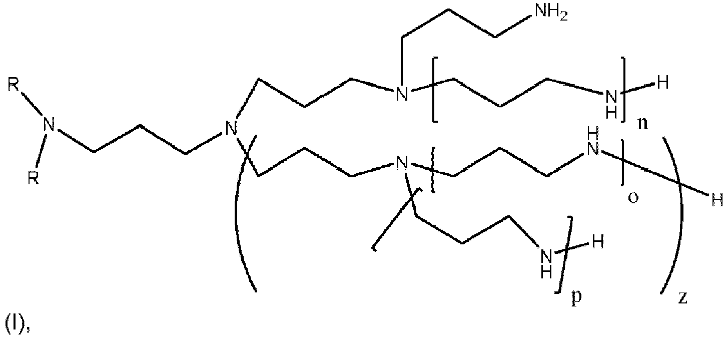 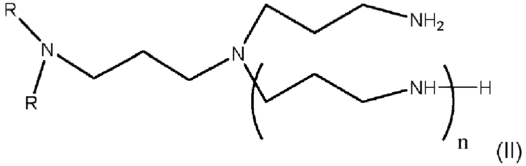 dans lesquels chaque R est, indépendamment de l'autre R, une fraction alkyle ou alcényle ayant de 8 à 22 atomes de carbone, n et z sont indépendamment l'un de l'autre 0, 1, 2, ou 3, et lorsque z > 0, alors o et p sont indépendamment l'un de l'autre 0, 1, 2, ou 3, ou des dérivés méthylés et/ou alcoxylés de ceux-ci, moyennant quoi ledit mélange comprend a) un total d'au moins 3 % en poids de composés ramifiés de formule (I) avec au moins l'un de n et z ≥ 1 et des produits de formule (II) avec n ≥ 1, et b) un total d'au moins 5 % en poids de produits de formule (I) avec n et z = 0, et des composés de formule (II) avec n = 0.

2. Composition de polyalkylamines selon la revendication 1 comprenant un total d'au moins 4 % p/p de composés ramifiés de formule (I) avec au moins l'un de n et z > 0 et de composés de formule (II) avec n > 0.

3. Composition de polyalkylamines selon la revendication 1 ou 2, comprenant au moins 6 % en poids de produits des formules (I) et (II) avec une structure linéaire, n'étant 0 dans les formules (I) et (II) et z étant 0 dans la formule (I).

4. Composition de polyalkylamines selon l'une quelconque des revendications précédentes comprenant des dérivés de polyalkylamines de di-alkyle ou alcényle gras, dans laquelle les dérivés sont des alcoxylates qui sont facultativement méthylés.

5. Composition de polyalkylamines selon l'une quelconque des revendications précédentes, comprenant des dérivés de polyalkylamines de di-alkyle ou alcényle gras, dans laquelle les dérivés sont méthylés.

6. Procédé de préparation des compositions de polyalkylamines selon l'une quelconque des revendications précédentes dans lequel une alkyldiamine de di-alkyle ou alcényle gras est mise en réaction dans deux cycles ou plus moyennant quoi chaque cycle comprend une étape de cyanoéthylation et une étape d'hydrogénation subséquente.

7. Procédé selon la revendication 6, dans lequel un catalyseur acide est utilisé durant les étapes de cyanoéthylation.

8. Procédé selon la revendication 6 ou 7, dans lequel la température de réaction durant une étape de cyanoéthylation ultérieure est plus élevée que la température durant une étape de cyanoéthylation antérieure.

9. Procédé selon l'une quelconque des revendications 6 à 8 dans lequel plus d'une mole d'acrylonitrile est utilisée par mole de la polyamine de départ.

10. Procédé de préparation des compositions de polyalkylamines selon l'une quelconque des revendications 1 à 5, dans lequel une alkyldiamine de di-alkyle ou alcényle gras est soumise à une étape de cyanoéthylation et à une étape d'hydrogénation subséquente, moyennant quoi dans l'étape de cyanoéthylation au moins 2 moles d'acrylonitrile sont utilisées par mole d'alkyldiamine de di-alkyle ou alcényle gras et un catalyseur acide est utilisé.

11. Procédé selon la revendication 10 dans lequel le produit est soumis à un ou plusieurs cycles supplémentaires comprenant une étape de cyanoéthylation et d'hydrogénation subséquente.

12. Procédé selon l'une quelconque des revendications 6 à 11 dans lequel un ou plusieurs solvants sélectionnés parmi un alcool en C₁₋₄ et des diols en C₂₋₄ sont présents dans l'étape de cyanoéthylation.

13. Procédé selon la revendication 12 dans lequel la quantité des solvants est de 0,1 à 50 % en poids du mélange réactionnel liquide.

14. Procédé de préparation des dérivés selon la revendication 4 ou 5, étant le procédé selon l'une quelconque des revendications 6 à 13, avec une étape supplémentaire dans lequel les polyalkylamines de di-alkyle ou alcényle gras sont méthylées, alcoxylées, ou les deux.

15. Utilisation des compositions de polyalkylamines de di-alkyle ou alcényle gras selon l'une quelconque des revendications 1 à 5 en tant que désémulsifiants pour des émulsions huile-dans-eau, inhibiteur de la corrosion, additif pour carburant, agent antitartre, additif pour l'asphalte, agent de récupération améliorée de pétrole pour les puits de pétrole, additif pour huile de coupe, et agent antistatique.
